(19) Europäisches Patentamt
European Patent Office
Office européen des brevets

(11) Numéro de publication: **0 333 645 B1**

(12) **FASCICULE DE BREVET EUROPEEN**

(45) Date de publication du fascicule du brevet: **09.01.91**

(51) Int. Cl.[5]: **C 07 C 271/06,** B 41 M 5/124

(21) Numéro de dépôt: **89810104.3**

(22) Date de dépôt: **08.02.89**

(54) **Encre désensibilisante pour impression d'une feuille autocopiante.**

(30) Priorité: **16.02.88 CH 553/88**

(43) Date de publication de la demande:
**20.09.89 Bulletin 89/38**

(45) Mention de la délivrance du brevet:
**09.01.91 Bulletin 91/02**

(84) Etats contractants désignés:
**AT BE CH DE ES FR GB GR IT LI NL SE**

(56) Documents cités:
**EP-A-0 119 958**
**FR-A-2 163 478**
**FR-A-2 206 711**
**GB-A- 761 107**
**US-A-3 105 063**
**US-A-3 658 882**
**US-A-3 872 150**

(73) Titulaire: **SICPA HOLDING S.A.**
**Burgstrasse 17**
**CH-8750 Glarus (CH)**

(72) Inventeur: **Amon, Albert**
**26, Avenue C.-Secrétan**
**CH-1005 Lausanne (CH)**
Inventeur: **Boksanyi, Laszlo Karoly**
**ch. Creux de Corsy 53**
**CH-1093 La Conversion (CH)**
Inventeur: **Degott, Pierre**
**Chemin de Margerol 3**
**CH-1009 Pully (CH)**

(74) Mandataire: **Mohnhaupt, Dietrich et al**
**AMMANN PATENTANWAELTE AG BERN**
**Schwarztorstrasse 31**
**CH-3001 Bern (CH)**

**EP 0 333 645 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention a pour objet une encre désensibilisante pour impression en offset sec ou humide, en typographie ou en flexographie sur une surface d'un ensemble autocopiant chimique comprenant au moins deux feuilles superposées dont les faces en regard présentent l'une un enduit électrophile et l'autre un enduit nucléophile, susceptibles de produire une réaction chromogène sur l'application d'une pression locale.

L'invention a également pour objet une nouvelle classe de composés chimiques nucléophiles destinés à l'usage comme agent actif d'une encre désensiblisante ainsi qu'un procédé de leur préparation.

On connaît déjà des ensembles autocopiants chimiques du genre mentionné ci-dessus où l'enduit nucléophile contient des microcapsules qui renferment un leucocolorant nucléophile. Les microcapsules éclatent sous l'effet de la frappe d'une machine à écrire ou de la pression d'un instrument d'écriture sur le recto de la première feuille, et une réaction colorée se produit entre le leuco-colorant nucléophile et un accepteur électrophile sur le recto de la deuxième feuille. De cette manière, on obtient la reproduction sur la deuxième feuille de l'écriture effectué sur la première feuille.

Il existe certains cas où l'on veut empêcher qu'une telle réaction ait lieu sur certaines parties de la surface de la deuxième feuille. Dans ce but, on neutralise les parties désirées de la surface acceptrice par impression d'un couche d'encre nucléophile.

Les principaux procédés d'impression qu'on peut utiliser pour appliquer l'encre neutralisante, donc l'encre désensibilisante, sur le papier autocopiant, sont le procédé offset sec et le procédé offset humide ou lithographique, le procédé flexographique et le procédé typographique.

Le procédé d'impression en offset sec utilise une plaque en élastomère où les surfaces à imprimer sont en surélévation. Ces surfaces sont enduites d'encre qui est transférée sur un blanchet en caoutchouc qui à son tour dépose l'encre sur la feuille à imprimer.

Ce procédé nécessite assez peu de pression pour assurer le transfert de l'encre sur le recto d'une feuille présentant au verso des microcapsules. Ainsi, le risque de faire éclater les microcapsules est assez faible.

Le procédé d'impression en offset humide ou lithographique utilise une plaque métallique où les zones à imprimer sont oléophiles et les zones ne devant pas être imprimées présentent un caractère hydrophile. Un encrier suivi d'un train d'encrage nourrit les zones oléophiles alors que des rouleaux mouilleurs humidifient les zones hydrophiles à partir d'un bac d'eau de mouillage. L'encre sur les zones oléophiles de la plaque est ensuite transférée sur un blanchet en caoutchouc pour finalement être déposée sur la surface à imprimer.

Au niveau de la plaque un équilibre des apports d'encre et d'eau doit s'établir en plus de l'équilibre d'émulsion possible de l'eau dans l'encre. Ces équilibres assurent la netteté d'impression et ils sont en relation physico-chimique avec l'équilibre hydrophile-lipophile (HLB).

Le procédé flexographique fait usage de formes en relief, constituées de cliches souples en caoutchouc ou en matière synthétique élastique disposés sur un rouleau d'impression. De façon similaire, le procédé typographique utilise une matrice contenant des surfaces surélevées. Les parties en relief des deux systèmes d'impression seront encrées et transmettent ensuite l'encre au substrat à imprimer.

Les procédés d'impression mentionnées sont d'ailleurs bien connus de l'homme du métier, jusque dans leurs moindres leurs détails, et ne seront donc pas répétés ici.

Les encres désensibilisantes connues, aussi appelées encres neutralisantes, sont destinées à être imprimées par offset sur certaines parties prédéterminées de la feuille acceptrice d'un ensemble autocopiant chimique où la feuille ou les feuilles comportent déjà au moins la couche acceptrice électrophile au recto. Nécessairement, l'encre désensibilisante doit avoir un caractère nucléophile pour qu'elle soit apte à neutraliser l'effet électrophile de la composition acceptrice.

De nombreux composés organiques ont déjà été proposés et utilisés comme composant actif nucléophile des encres désensibilisantes, aptes à neutraliser aux endroits prédéterminés le composant électrophile de la couche acceptrice, afin d'empêcher la formation d'une couleur sous l'effet de la pression qui écrase les microcapsules contenant le leucocolorant nucléophile. Ces composants actifs nucléophiles sont choisis en fonction du procédé d'impression et, dès lors, de la composition de l'encre d'impression. Le choix est limité car le composé actif doit remplir une pluralité de conditions bien connues de l'homme du métier.

En général, le propriété nucléophile du composant actif est basée sur la présence de paires d'électrons libres sur des atomes électronégatifs, en particulier l'oxygène et l'azote, aptes à se combiner avec une lacune de paires d'électrons sur un atome électropositif donc électrophile tel que carbone, soufre, bore, etc.

Comme composant nucléophile actif des encres désensibilisantes, on a déjà proposé les polyalkylèneglycols, la glycérine, des sels d'ammonium quaternaires à longue chaîne grasse, et des amines à longue chaîne. Ces composés désensibilisantes présentent un certain nombre d'inconvénients. Par exemple l'effet de désensibilisation peut diminuer sous l'influence de chaleur, d'humidité ou de lumière. D'autres composés ont tendance de migrer sur des parties de la couche acceptrice où une coloration doit se produire. La plupart des composés connus sont sujets à un brunissement après un certain temps. Les amines et leurs dérivés simples ont en général une odeur forte et désagréable; certains entre eux sont toxiques ou allergènes.

On a essayé de pallier ces inconvénients en proposant d'autre composés nucléophiles actifs. Par

exemple, la publication EP—AI—0 119 958 décrit des encres désensibilisantes contenant un composé nucléophile alcanoxylé qui est ponté par exemple par réaction avec un polyisocyanate. On mentionne également des amines alcoxylées comme composés de départ de la réaction avec le polyisocyanate mais, la fonctionnalité hydroxyle de ces composés n'étant pas réduite auparavant, des polymérisations incontrôlables se produisent empêchant un définition nette du produit ponté. La demande de brevet européen publiée no 0 088 466 décrit un composé actif nucléophile qui est seulement un dérivé alcoxylé d'un composé organique ayant un atome d'hydrogène actif, tel qu'alcool, phénol, acide gras ou amine, polyalcoxylé au moyen d'oxyde d'éthylène et/ou d'oxyde de propylène. Le brevet DE—C3—25 26 592 décrit des amines polyalcoxylées mais qui sont au moins partiellement estérifées ou étherifiées sur les groupes hydroxyle terminaux provenant de l'alcoxylation des mono- et polyamines.

Cependant, bien que les composés neutralisants selon le brevet allemand DE—C3—25 26 592 apportent une amélioration au point de vue odeur de hydrophobie des composés, il a été constaté que le pouvoir neutralisant ou désensibilisant de ces composés est comparable, voire plus faible, par rapport à celui des composés connus ou à ceux qui se trouvent sur la marché comme composant d'une encre désensibilisante, ce qui implique de déposer une quantité d'encre importante pour assurer une désensibilisation parfaite.

Or l'utilisation de l'encre désensibilisante présente en elle-même des inconvénients. D'abord, le séchage d'une encre est d'autant plus long que la quantité d'encre déposée est importante, et le temps de séchage est une fonction exponentielle et non linéaire de l'épaisseur. Ce fait est d'une importance primordiale car, si la vitesse de séchage est trop lente, on observe la maculage des feuilles imprimées qui sont déposées en pile en sortant de l'imprimeuse, ou le maculage des couches successives lorsqu'on enroule le papier en bobine.

Ensuite, du point de vue économique, le coût total d'une impression désensibilisante est dû pour une part importante au coût de l'encre.

Il existe donc un besoin urgent d'encres désensibilisantes qui ont un pouvoir neutralisant accru afin de réduire la quantité d'encre à imprimer par unité de surface pour arriver à la neutralisation complète de la couche acceptrice électrophile sous-jacente, et afin d'assurer un séchage rapide.

L'invention a donc pour but de développer une nouvelle encre désensibilisante contenant comme principe actif nucléophile de nouveaux composés aptes à neutraliser les composants électrophiles, encre qui sera imprimée en offset, en typographie ou un flexographie et qui est apte à désensibliser une couche électrophile d'un ensemble autocopiant chimique. Ces composés se distinguent par un pouvoir neutralisant accru par rapport aux composés connus, tout en présentant les mêmes propriétés avantageuses ou nécessaires des composés connus. Vu le prix élevé des composés désensibilisants en général, une augmentation du pouvoir neutralisant de 25% constituerait déjà un très grand avantage et permettrait de diminuer en conséquence la quantité d'encre déposée et le temps de séchage.

L'encre désensibilisante selon l'invention est définie dans la revendication indépendante 1; des exécutions spéciales font l'objet des revendications dépendantes 2 à 5.

Un autre but de l'invention est de développer une nouvelle classe de composés destinés à être utilisés dans les encres désensibilisantes pour offset, typographie ou flexographie contenant ces nouveaux composés nucléophiles, et encore un procédé de préparation de ces composés.

Ces composés, présents dans les encres de l'invention et définis dans la revendication indépendante 6, sont donc des produits d'addition de mono, di ou polyisocyanates avec des dérivés éthoxylés et/ou propoxylés d'une amine, diamine ou polyamine primaire ou secondaire ou d'une alcanolamine, et dont la fonctionnalité hydroxyle subséquent à l'éthoxylation (et/ou la propoxylation) a été réduite à 0,5 à 2 par éthérification, estérification or uréthanisation.

Le composé aminé de base peut être choisi parmi les composés suivants:

amines primaires, diamines primaires ou polyamines primaires (p.e. polyéthylène imine)

amines secondaires, diamines secondaires ou polyamines secondaires, et

alcanolamines qui peuvent être primaires, secondaires ou tertiaires,

mais suivant une disposition préférée est plutôt choisi parmi les amines et diamines primaires et secondaires.

Ces composés aminés sont éthoxylés et/ou propoxylés par les méthodes connues via l'addition des groupes aminés et/ou des groupes hydroxyles sur la fonction oxirane de l'oxyde d'éthylène et/ou l'oxyde de propylène. L'éthoxylation et/ou la propoxylation peut être poursuivie via l'addition des groupements hydroxyles créés lors de l'ouverture des cycles oxirane sur des entités oxirane présentes en excès, le contenu final du composé en groupements alcoxy étant régi par le rapport massique entre le composé aminé et l'oxyde d'éthylène (et/ou l'oxyde de propylène). La fonctionnalité hydroxyle du composé, définie comme le nombre de groupements hydroxyles terminaux par molécule de produit intermédiaire, est égale au nombre d'hydrogènes actifs présents sur le composé aminé de base; elle peut varier entre 1 chez l'amine secondaire éthoxylée et 4, par example diamine primaire éthoxylée si les composés aminés sont choisis parmi les amines et diamines primaires ou secondaires, les alcanolamines primaires, secondaires ou tertiaires et peut être plus grande dans le cas de polyamines primaires ou secondaires.

Ces composés alcoxylés en mélange ou non sont alors uréthanisés par addition avec un mono, di ou polyisocyanate.

Il est important de veiller à ce que la fonctionnalité hydroxyle moyenne du produit alcoxylé ou du

mélange de produits alcoxylés soit inférieure à 2 et préférablement proche de 1. Dans le cas inverse, il est difficile de contrôler la viscosité finale du produit ou même parfois d'éviter la gélification, ou la quantité de di ou polyisocyanate qui peut être ajoutée est trop faible pour que l'uréthanisation apporte les avantages souhaités.

Il est donc nécessaire d'abaisser la fonctionnalité hydroxyle moyenne des produits intermédiaires alcoxylés avant uréthanisation. Pour ce faire, une alternative consiste à mélanger entre eux des produits alcoxylés de fonctionnalité hydroxyle différente de telle sorte qui le mélange ainsi réalisé comporte une fonctionnalité hydroxyle moyenne inférieure à deux, les produits alcoxylés de fonctionnalité hydroxyle inférieure à 2 introduits lors du mélange jouant alors la rôle de limitateurs de chaîne lors de la polyaddition avec les di ou polyisocyanates.

Une autre alternative consiste à abaisser la fonctionnalité hydroxyle d'un produit aminé alcoxylé par réaction d'une partie ou de la totalité des groupes hydroxyles avec un groupement monofonctionnel réactif vis-à-vis d'eux jusqu'à ce que la fonctionnalité hydroxyle moyenne de la molécule ainsi produite soit inférieure à 2, préférablement proche de 1.

Les réactions chimiques possibles sont l'éthérification, l'estérification et l'uréthanisation. Les composés monofonctionnels utilisables sont respectivement des halogènures d'alkyle ou de cycloalkyle, des acides carboxyliques ou sulfoniques et leurs dérivés fonctionnels et des monoisocyanates.

Il est possible d'utiliser successivement les réactions mentionnées aux fins de l'invention, p.e. d'éthérifier jusqu'à un certain point les composés aminés alcoxylés, puis de les estérifier jusqu'à obtenir la fonctionnalité hydroxyle souhaitée. Certaines précautions doivent cependant être prises pour éviter une réaction parasite, par exemple entre un agent d'estérification non totalement réagi avec un monoisocyanate, mais ces techniques sont connues en elles-mêmes.

Il est également possible de mélanger entre eux les produits aminés alcoxylés traités par ces techniques, ou de mélanger ces mêmes produits avec des produits aminés alcoxylés non traités de manière à obtenir la fonctionnalité hydroxyle moyenne désirée avant le traitement avec un di ou polyisocyanate.

Les mono, di et polyisocyanate utilisés pour l'uréthanisation finale sont des produits disponibles sur le marché. D'après une exécution préférée de l'invention, un diisocyanate de structure OCN—R—NCO où R est un radical bivalent aliphatique ou cycloaliphatique (p.e. hexaméthylène) ou aromatique (p.e. phénylène, tolylène ou naphtylène) est particulièrement adéquat.

La quantité d'équivalents isocyanates utilisés pour la réaction d'uréthanisation est proche de la quantité d'équivalents hydroxyles du produit intermédiaire, préférablement légèrement inférieure, de telle sorte que pour un groupe hydroxyle libre, il y a formation d'un groupement uréthane ou légèrement moins. L'indice hydroxyle final du produit uréthanisé, exprimé en mg KOH par g de produit, est généralement inférieur à 60, préférablement inférieur à 20.

Une classe importante des nouveaux composés neutralisants a la structure générale suivante:

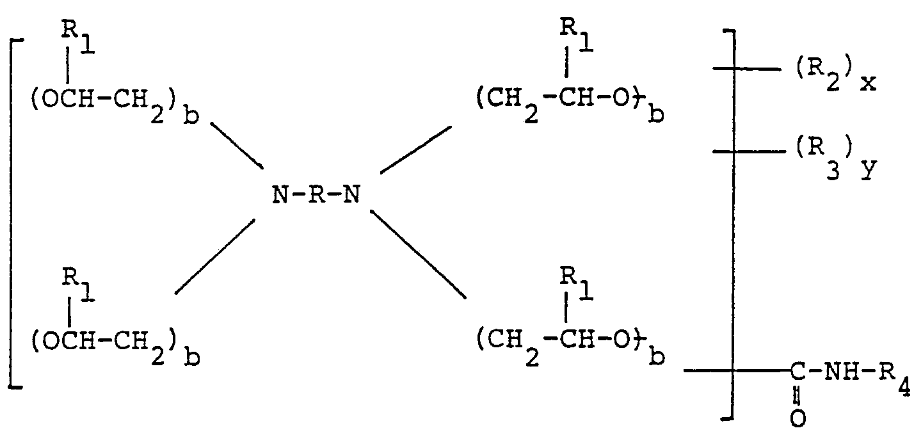

(II)

dans laquelle:

R représente un radical aliphatique ou aromatique bivalent,

$R_1$ est de l'hydrogène ou un groupe méthyle,

$R_2$ est un groupe alkyle, de préférence alkyle inférieur,

$R_3$ le radical acyle d'un acide carboxylique en $C_2$ à $C_{22}$,

$R_4$ est un groupement organique pouvant comporter au moins un substituant de formule

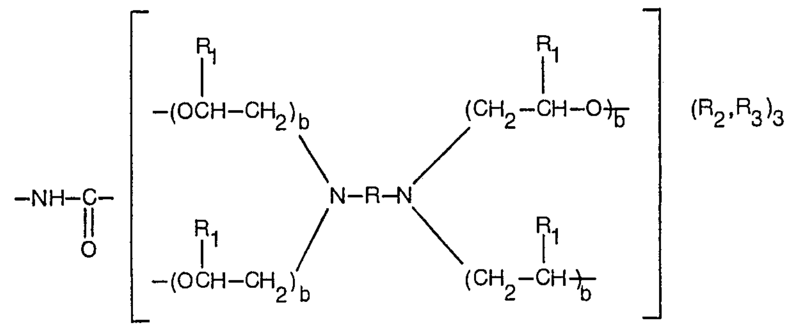

et

b est un nombre compris entre 1 et 6.

Dans la formule générale (II), R est de préférence un groupement de formule $—(CH_2)—_i$ où i est un nombre de 2 à 12; le reste

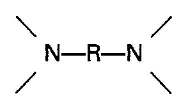

est donc celui d'éthlènediamine, de propylènediamine, de butylènediamine, de pentylènediamine, d'hexylènediamine jusqu'à la dodécylènediamine. R peut aussi être un radical bivalent aromatique tel que phénylène, toluylène ou naphtylène.

Les symboles $R_1$ désignent indépendamment un atome d'hydrogène ou un groupement méthyle.

$R_2$ dans la formule (II) peut également représenter le radical acyle d'un acide carboxylique en $C_2$ à $C_{22}$. Les radicaux acyle inférieur, tels qu'acétyle, propionyle, butyryle et leurs dérivés substitués sont préférés lorsqu'il ne s'agit que de bloquer des groupes hydroxyle libres. D'autre part, on préfères les radicaux acyle gras, mentionnés ci-dessus et leurs dérivés simples, lorsqu'on veut augmenter la solubilité des produits finals de formule (II) dans les huiles, donc l'hydrophobie.

Si l'on utilise des radicaux acyule substitués, on veillera à ce que les substituants n'interféront pas avec la réaction subséquente de formation de la liaison uréthane. Par exemple, le substituant hydroxy est à éviter tandis que les halogènes ou les méthoxy sont acceptables.

Les composés neutralisants décrits ci-dessus peuvent être préparés en plusieurs étapes, par des techniques connues en elles-mêmes.

La première étape est l'alcoxylation d'une mono, di, polyamine ou alcanolamine. Les méthodes connues seront appliquées. Par exemple on peut mélanger l'entité aminée avec l'oxyde d'éthylène et/ou de propylène, éventuellement en ajoutant un solvant, mais en observant des conditions anhydres, et en chauffant le mélange. On peut également ajouter l'oxyde d'éthylène et/ou de propylène, éventuellement sous pression, à la mono, di, polyamine ou alcanolamine à température élevée, en présence ou non d'un solvant, dans des conditions anhydres. Un catalyseur tel qu'un acide ou une base peut être ajouté. Les quantités molaires d'oxyde d'éthylène ou de propylène qui s'additionnent sont déterminées par les quantités employées d'oxyde d'éthylène et/ou de propylène. On choisit ces quantités telles que la masse moléculaire du produit d'addition ainsi obtenu soit comprise entre 100 et 10000 g/mole.

La seconde étape du procédé de préparation comprend l'éthérification, l'estérification ou l'uréthanisation avec un composé monofonctionnel réactif vis-à-vis des groupements hydroxyles, ces réactions visant principalement à abaisser la fonctionnalité hydroxyle moyenne des composés intermédiaires. Cette étape n'est pas indispensable si le produit aminé alcoxylé ou le mélange de tels produits a une fonctionnalité hydroxyle moyenne inférieure à 2.

L'éthérification est effectuée avec des réactifs introduisant un groupement alkyle inférieur tel que méthyle, éthyle, propyle, i-propyle, butyle, i-butyle ou tert-butyl. Dans cette étape, on applique les méthodes connues d'éthérification. Par exemple, on peut fair réagir le produit intermédiaire alcoxylé dont l'isolation du mélange réactionnel de l'alkoxylation n'est pas nécessaire, avec un halogénure d'alkyle de formule $R_2—Y$ où $R_2$ à la signification donnée ci-dessus, et Y est un atome d'iode, de brome ou de chlore, ou avec un autre composé analogue où Y est un groupe partant tel que sulfate ou tosyle. On préfère $R_2$ = butyle et Y = brome. On travaille généralement à température élevée, en l'absence d'eau, au sein d'un solvant et evéntuellement après avoir fait réagir le produit intermédiaire avec un métal alcalin tel que sodium ou potassium.

L'estérification est un technique également connue en soi. Les réactifs utilisés sont des acides carboxyliques ou sulfoniques ou leurs dérivés réactifs comme leurs halogénures, leurs anhydrides ou certains de leurs esters. Les conditions de réactions sont variables selon le type d'agent d'acylation utilisé. Généralement un composant basique est utilisé dans le cas d'halogénures d'acides, alors qu'un sel d'étain

ou un acide fort est un cataylseur efficace dans le cas d'une estérification effectué avec l'acide proprement dit. Dans ce dernier cas, il faut évacuer l'eau issue de la réaction au fur et à mesure de sa formation.

L'uréthanisation avec un monoisocyanate est également une technique connue en soi. Les détails concernant cette méthode seront données plus loin.

Les trois techniques mentionnées peuvent être utilisées seules ou successivement dans n'importe quel ordre, mais préférablement en effectuant d'abord l'éthérification.

Cette réaction nécessite en effet des temps de réaction très longs, alors que certains dérivés réactifs d'acides carboxyliques ou sulfoniques, comme leurs halogénures, ou les monoisocyanates sont des composés très réactifs vis-à-vis des groupes hydroxyles.

Pour des raisons économiques, il es donc préféré d'éthérifier seulement une partie des groupes hydroxyles libres et d'amener ensuite le produit étherifié à la fonctionnalité hydroxyle moyenne désirée par estérification ou uréthanisation avec un dérivé réactif d'un acide carboxylique ou sulfonique ou un monoisocyanate.

Le produit de réaction de cette seconde étape peut être préparé, par exemple, selon le brevet allemand DE—C3—25 26 592. Ce brevet décrit la préparation de composés neutralisants pour encres désensibilisantes qui sont obtenus (1) par l'addition d'oxyde d'éthylène et/ou de propylène sur l'ammoniaque, les amines alkyliques, alkyléniques, alkényliques, aryliques ou cycliques, jusqu'à l'épuisement de l'hydrogène actif, et (2) éthérification d'au moins 25%, de préférence 50% ou d'avantage des groupes hydroxyle terminaux avec un halogénure d'alkyle.

Bien qu'une fonctionnalité hydroxyle moyenne proche de 1, préférée par cette invention, ne soit pas décrité, le mode préparatoire décrit dans la publication citée peut être appliqué.

Le produit de réaction de cette seconde étape est donc un éther, un ester ou un monouréthane de fonctionnalité hydroxyle moyenne comprise entre 0,5 et 2 et préférablement proche de 1. Ces trois fonctionnalités pouvant être présentes seules ou en combinaison. Ceci équivaut à dire que la plus grande partie des molécules comportent 1 ou 2 groupes hydroxyles libres, mais n'exclut pas qu'une fraction minime de ces mêmes molécules ne comportent plus aucun groupe hydroxyle ou en comportent 3 voire plus.

Pour les besoins de l'invention,l il n'est pas nécessaire de fractionner le mélange pour isoler l'espèce de fonctionnalité hydroxyle désirée.

Dans le procédé de l'invention, on effectue alors une uréthanisation du produit issu de cette deuxième étape, d'un mélange de ces produits, de produits issus de la première étape, d'un mélange de ces produits, ou d'un mélange de produits issus de la première et de la seconde étape, le point important étant de veiller à ce que la fonctionnalité hydroxyle moyenne de ces produits ou de ces mélanges soit supérieure à 0,5 et inférieure à deux pour pouvoir introduire une quantité suffisante d'isocyanates afin d'en obtenir les avantages recherchés tout en évitant la gélification en contrôlant la masse moléculaire et la viscosité du produit final.

L'uréthanisation est effectué avec un mono, di ou polyisocyanate ou un mélange de ces composés, préférablement un diisocyanate, dans une proportion molaire isocyanate/hydroxyle proche de 1, préférablement légèrement inférieure, typiquement 0,9:1.

L'indice hydroxyle du produit final est généralement inférieur à 60 mg KOH/g, préférablement inférieur à 20 mg KOH/g.

L'uréthanisation est effectuée à température moyenne, comprise entre 60 et 150°C, avec ou sans catalyseur surajouté, les amines tertiaires présentes dans le mélange traité étant de bons catalyseurs d'uréthanisation.

La réaction peut être conduite en l'absence de solvant ce qui supprime la nécessité d'isoler le produit. Cependant puisque la formation d'uréthanes à partir d'isocyanates est exothermique un solvant inerte vis-à-vis des différents réactifs, tel qu'un hydrocarbure, peut être employé pour dissiper la chaleur de réaction. L'augmentation de température peut d'ailleurs être contrôlée par le biais de la vitesse d'adjonction de l'isocyanate. Finalement le produit uréthanisé est isolé du mélange réactionnel et purifié si nécessaire.

Le nouveau produit selon l'invention est liquide à température ambiante, a une viscosité moyenne, est pratiquement incolore, sans odeur et chimiquement stable. Il n'est pas toxique lorsqu'il est exempt d'isocyanate résiduel ce qui est le cas lorsque la stoechiométrie indiquée est respectée, et qui peut d'ailleurs être facilement éliminé par réaction subséquente avec un monoalcool, par exemple l'isopropanol.

Le produit selon l'invention a d'excellentes propriétés neutralisantes lorsqu'il est incorporé dans les encres désensibilisantes. Il est utilisable pour encres offset humide et offset sec, en typographie ou en flexographie.

Le nouveau composé peut être utilisé dans les encres désensibilisantes sous la forme d'une seule espèce ou en mélange de deux ou plusieurs représentants.

Ce ne sont que des exemples, et l'homme du métier comprendra qu'on peut employer tous les produits de départ en mélange, ou une combinaison des produits obtenus, afin d'adapter le composé actif neutralisant aux besoins de la pratique.

Preparation du compose selon l'invention

Exemple 1

Dans un réacteur hermétique équipé d'un système de chauffage, d'un agitateur mécanique, d'un thermomètre, et d'un entonnoir à robinet (ampoule à brome), on place 90 parties en poids d'une substance liquide de formule

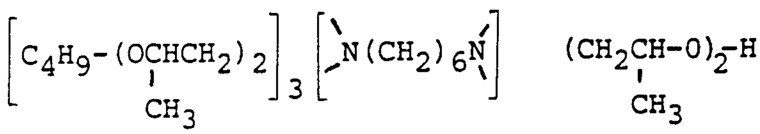

ayant une fonctionnalité hydroxyle de 1, obtenue selon les modes opératoires décrits ci-dessus. Ce produit est pratiquement incolore et présente un indice hydroxyle de 79, exprimé comme d'habitude en mg KOH/g.

On porte le contenue du récipient à 80°C, et l'on introduit sous agitation goutte à goutte 10 parties en poids de diisocyanate de toluène (TDI), mélange d'isomères. Le TDI est un produit commercialisé.

En l'espace d'une heure, l'addition de TDI est terminée. La température monte à 95°C lors de l'introduction de TDI. Elle est ensuite fixée à 120°C pendant le temps de la réaction.

Les quantités indiquées de réactifs sont choisies pour établir un rapport d'équivalents OH/NCO de 1,1 ce qui correspond à un rapport molaire hydroxyéther/diisocyanate de 2,1:1.

Après refroidissement, on obtient un liquide presque incolore ayant une masse moléculaire moyenne en poids de 2600 ± 200, une masse moléculaire moyenne en nombre de 1100 ± 80, un viscosité de 2,5 Pa.s à 25°C, une teneur en azote de 5,2 ± 0,1% et un indice hydroxyle de 19 environ (exprimé en mg KOH/g).

Le produit final a la formule

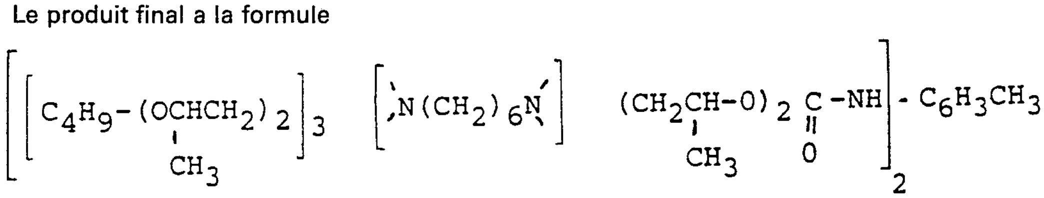

(IV)

(formule II; $R = (CH_2)_6$; $R_1 = CH_3$; $R_2$ = n-butyle; b = 2; x = 3; y = 0; $R_4$ = toluylène substitué par une entité du groupe en grands crochets).

Exemple 2

On dissout 90 parties en poids du produit de formule I selon l'exemple 1, dans 67 parties en poids de xylène, dans l'installation décrite à l'exemple 1. On porte la solution à 75°C, et l'on ajoute sous agitation et goutte à goutte, 10 parties en poids de TDI. La température monte à 85°C lors de l'adjonction du TDI puis est maintenue à 110°C par chauffage adequat. Cette réaction est terminée après 1h 30.

Le xylène est éliminé par distillation sous vide à 110°C.

Le produit obtenu comme résidu est en tous points identique à celui de l'exemple 1.

Exemple 3

L'exemple 1 est répété mais en utilisant 92 parties en poids du composé de formule indiquée et 8 parties en poids du TDI.

La température à laquelle le TDI est ajouté est de 80°C, la température de réaction 100°C, et la durée de réaction 1 heure.

Les quantités employées de réactifs correspondent à un rapport d'équivalents OH/NCO de 1,4 et à un rapport molaire hydroxyéther/TDI de 2,9:1.

Le produit présente les caractéristiques suivantes: indice hydroxyle 21; masse moléculaire moyenne pondérale 2000 ± 100, masse moléculaire moyenne numérique 850 ± 50, teneur en azote 4,9 ± 0,1%.

Exemple 4

On dissout 100 parties en poids d'un produit de formule moyenne

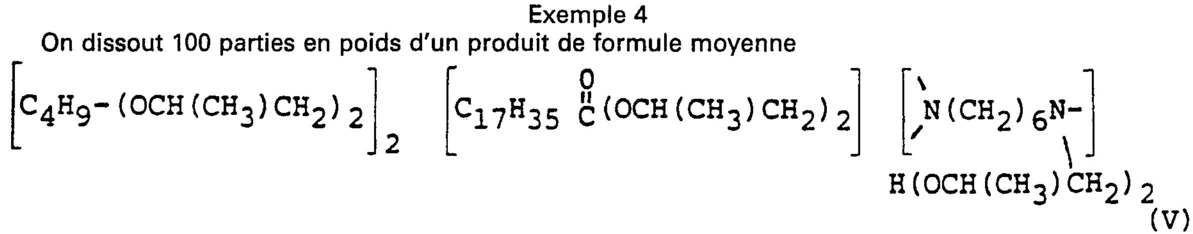

(V)

(formule III; $R = (CH_2)_6$; $R_1 = CH_3$; b = 2; x = 2; $R_2 = C_4H_9$ (n-butyle); y = 1;

$R_3$ = 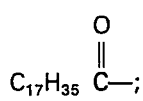;

x et y étant repris de la formule II) dans 75 parties en poids de xylène, dans l'installation décrite à l'Exemple 1. On porte la solution à 70°C, et l'on ajoute sous agitation et goutte à goutte, 89,5 parties en poids de tolylène-diisocyanate.

La suite des opérations est indentique à celle de l'Exemple 2.

On obtient un produit légèrement jaunâtre ayant un indice hydroxyle de 20 mg KOH/g environ et un excellent pouvoir solvent vis-à-vis de composés oléosolubles.

Le produit de départ de formule V a été obtenu par éthérification puis estérification, en utilisant successivement le bromure de butyle et le chlorure de stéaroyle en milieu basique pour la réaction avec le composé de fonctionnalité hydroxyle 4 de formule

$$\left[\,>N-(CH_2)_6-N<\,\right]\left[(-CH_2\overset{CH_3}{\overset{|}{C}H}O)_2\ H\right]_4$$

Exemple 5

Le produit

$$N(CH_2—CH_2—O)_3[(CH_2—CH_2—O)\overset{O}{\overset{\|}{C}}—C_{11}H_{23}]_2[CH_2—CH_2—O)H]\ \text{où}\ N(CH_2—CH_2—O—)_3$$

est le reste de triéthanol amine, obtenu par estérification du polyalcool correspondant avec le chlorure de lauroyle en milieu basique, jusqu'à obtenir une fonctionnalité hydroxyle moyenne du produit voisine de 1, est uréthanisé avec l'hexaméthylène diisocyanate en présence d'un sel d'étain (dibutyl-dilaurate d'étain). Le rapport OH/NCO est fixé à 1,1 et la réaction est menée comme dans l'exemple 1, en l'absence de tout solvant.

Le produit obtenu est essentiellement un dimère du produit tel que décrit ci-dessus, comportant en moyenne un peu moins d'un groupement uréthane par entité aminée. L'indice hydroxyle du produit est voisin de 10 mg KOH/g. Sa couleur est jaune clair. Le produit présente un excellent pouvoir solvant vis-à-vis de composés oléosolubles et les propriétés désensibilisantes de l'encre, dont il est le principe actif, sont excellentes.

Exemple 6

Le produit de formule $N(CH_2—CH_2—O)_3[(CH_2CH_2O—)_2]_3H$ est mélangé au produit de formule

$$(C_2H_5)_2N(CH_2—\overset{CH_3}{\overset{|}{C}H}—O—)_2H$$

dans un rapport molaire 1:4; la fonctionnalité hydroxyle moyenne du mélange est égale à 1,4.

Le mélange est uréthanisé avec tolylène diisocyanate, le rapport OH/NCO étant fixé à 1,3, dans les mêmes conditions que celles décrits dans l'exemple 2, c'est-à-dire en présence de xylène. Une fois le solvant inerte tiré sous vide, on obtient un produit très clair, de viscosité moyenne, d'indice hydroxyle proche de 55 mg KOH/g. Le produit confère à l'encre, dont il est le principe actif, d'excellentes propriétés neutralisantes.

Preparation et utilisation d'une encre

Exemple 7

On prépare une encre désensibilisante offset en mélangeant intimément sur un moulin à trois cylindres les substances suivantes, les parties étant en poids:

| | |
|---|---|
| Produit de l'exemple 1 ou 2 | 60 parties |
| résine phénolique "Albertol KP 823" | 10 parties |
| dioxide de titane (pigment) | 15 parties |
| carbonate de calcium (pigment) | 8 parties |
| polypropylène glycol (régulateur de viscosité) | 7 parties |

Les deux premiers composants mentionnées ci-dessus sont prémélangés à chaud avant l'incorporation dans l'encre.

Une encre offset de comparaison, appartenant à l'état de la technique, est préparée de la même manière, mais le produit de l'exemple 1 ou 2 est remplacé par le même quantité d'un polypropylène glycol ayant la même viscosité.

On imprime par endroits le feuille acceptrice (CF) d'un ensemble autocopient chimique en papier, et on évalue le pouvoir neutralisant en frappant par une machine à écrire des croix sur le recto de la feuille comportant au verso des microcapsules (CFB).

La quantité de vernis imprimée est de 2 g/m² et de 2,8 g/m² pour chacune des deux encres.

On constate que les croix frappées sont visibles même aux endroits désensibilisés avec l'encre de comparaison, un peu moins fort lorsque 2,8 g/m² d'encre sont déposés, mais complètement invisibles aux endroits désensibilisés avec 2 et 2,8 g/m² d'encre selon l'invention.

On peut donc réduire la quantité d'encre désensibilisante par unité de surface, nécessaire pour désensibiliser de manière efficace la feuille réceptrice, d'au moins 40%.

L'homme du métier comprendra aisément qu'on peut incorporer les composés selon l'invention dans les encres pour flexographie et typographie. Les résultats après impression sont les mêmes que ceux qui vienneent d'être décrits.

**Revendications**

1. Encre désensibilisante pour l'impression en offset sec ou humide, en typographie ou en flexographie sur une surface d'un ensemble autocopiant chimique comprenant au moins deux feuilles superposées dont les faces en regard présentent l'une un enduit électrophile et l'autre un enduit nucléophile, susceptibles de produire une réaction chromogène sur l'application d'une pression locale, caractérisée en ce qu'elle contient, à titre d'agent actif désensibilisant, au moins un composé nucléophile étant le produit d'addition d'une mono, di ou polyisocyanate avec un dérivé éthoxylé et/ou propoxylé d'une mono, di ou polyamine primaire ou secondaire ou une alcanolamine et dont la fonctionnalité hydroxyle été réduite après l'alcoxylation à 0,5 à 2 par éthérification, estérification ou uréthanisation.

2. Encre selon la revendication 1, caractérisée en ce que le composé nucléophile répond à la formule

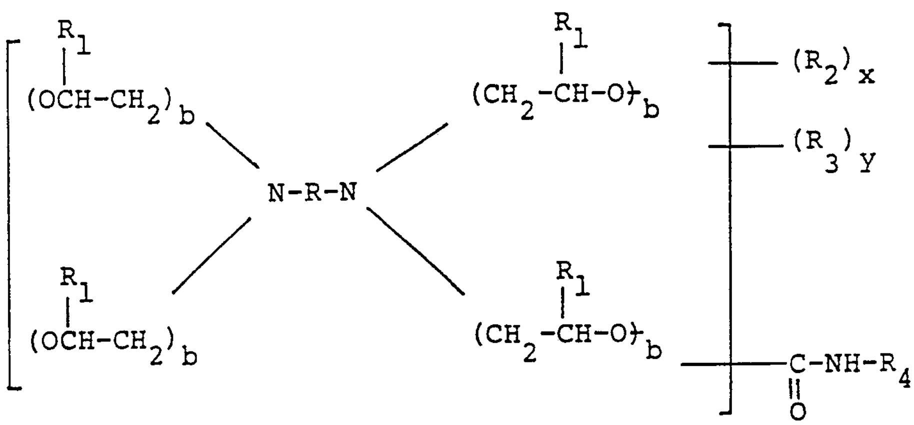

(II)

dans laquelle:

R représente un radical aliphatique ou aromatique bivalent,

$R_1$ est de l'hydrogène ou un groupe méthyle,

$R_2$ est un groupe alkyle,

$R_3$ est le radical acyle d'un acide carboxylique en $C_2$ à $C_{22}$,

$R_4$ est un groupement organique pouvant comporter un ou plusieurs substituants de formule

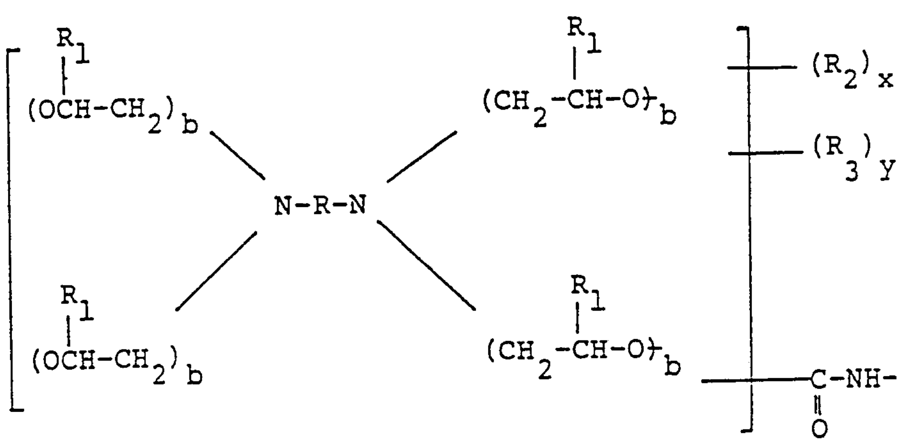

*x* + *y* étant 3, et

*b* est un nombre compris entre 1 et 6.

3. Encre selon la revendication 2, caractérisée en ce qu'elle contient un composé nucléophile de formule

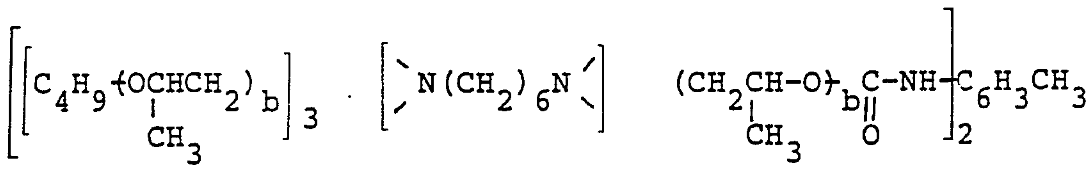

(IV)

où b est 2 ou 3.

4. Encre selon la revendication 2, caractérisée en ce qu'elle contient un composé de formule II dans laquelle R est un reste de formule $—(CH_2)_i—$ où i est un nombre de 2 à 12.

5. Encre selon la revendication 2, caractérisée en ce qu'elle contient un composé de formule II dans laquelle R est un radical bivalent aromatique.

6. Composé nucléophile, destiné à l'usage comme agent actif d'une encre désensibilisante, caractérisé en ce qu'il est le produit d'addition d'une mono, di ou polyisocyanate avec un dérivé éthoxylé et/ou propoxylé d'une mono, di ou polyamine primaire ou secondaire ou un alcanolamine, et dont la fonctionnalité hydroxyle a été réduite après alcoxylation à 0,5 à 2 par éthérification, estérification ou uréthanisation.

7. Composé nucléophile selon la revendication 6, caractérisé par la formule

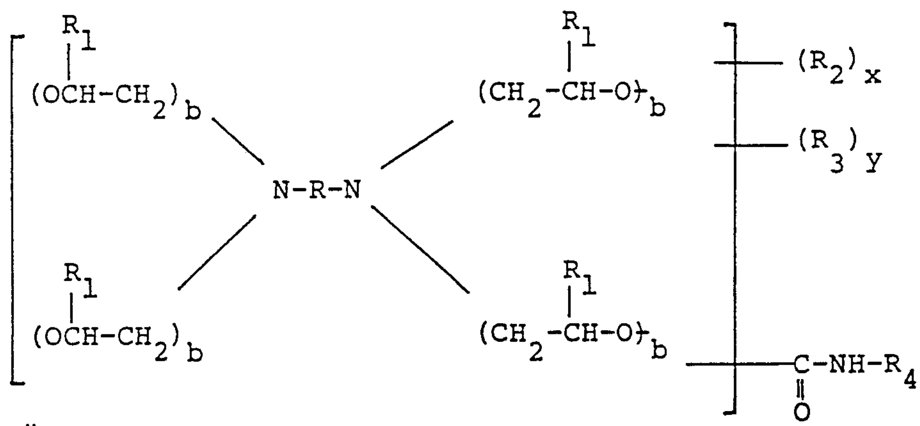

(II)

dans laquelle:

R représente un radical aliphatique ou aromatique bivalent,

$R_1$ est de l'hydrogène ou un groupe méthyle,

$R_2$ est un groupe alkyle,

$R_3$ est le radical acyle d'un acide carboxylique en $C_2$ à $C_{22}$,

$R_4$ est un groupement organique pouvant comporter un ou plusieurs substituants de formule

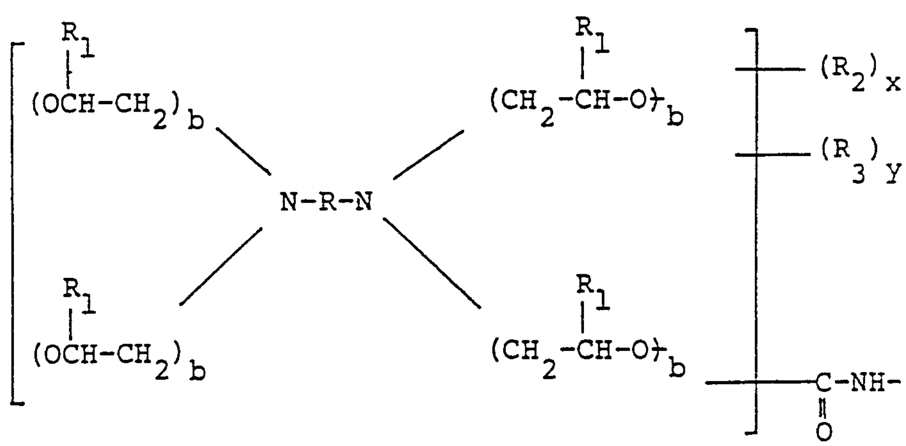

dans laquelle

*b* est un nombre compris entre 1 et 6, et

$x + y = 3$.

8. Composé selon la revendication 7, caractérisé en ce qu'il répond à la formule

$$\left[\left[C_4H_9(OCH(CH_3)CH_2)_b\right]_3 \left[>N(CH_2)_6N<\right] (CH_2CH(CH_3)-O)_b C(=O)-NH\right]_2 C_6H_3CH_3 \quad (IV)$$

où b est 2 ou 3.

9. Composé selon la revendication 7, caractérisé en ce que, dans la formule II, R signifie un reste de formule $-(CH_2)_i-$ où i est un nombre de 2 à 12.

10. Composé selon la revendication 7, caractérisé en ce que, dans la formule II, R est un radical bivalent aromatique.

11. Procédé de préparation d'un composé selon la revendication 6, caractérisé en ce qu'on soumet une mono, di ou polyamine primaire ou secondaire, ou un alcanolamine, à une éthoxylation et/ou propoxylation, qu'on réduit par éthérification et/ou acylation et/ou par uréthanisation avec un monoisocyanate, la fonctionnalité hydroxyle à 0,5 à 2, et qu'on fait réagir ce produit avec un mono, di ou polyisocyanate.

12. Procédé de préparation d'un composé de formule II selon la revendication 7, caractérisé en ce qu'on fait réagir un hydroxyéther ou hydroxyester, respectivement de formule III

$$\left[(R_2 \text{ ou } R_3)(OCH(R_1)-CH_2)_b\right]_3 \left[>N-R-N<\right] (CH_2-CH(R_1)-O)_b H \quad (III)$$

avec un mono, di ou polyisocyanate comportant le groupement $R_4$, la signification des symboles étant donnée à la revendication 7.

13. Procédé selon la revendication 12, caractérisé en ce que l'on utilise un diisocyanate $(OCN)_2R'$ où R' est un radical bivalent aromatique.

14. Procédé selon la revendication 12, caractérisé en ce qu'on fait réagir un éther partiel de formule

$$\left[C_4H_9(OCH(CH_3)CH_2)_b\right]_3 \left[>N(CH_2)_6N<\right] (CH_2CH(CH_3)-O)_b H$$

dans laquelle b est 2 ou 3, avec le toluylène diisocyanate.

## Patentansprüche

1. Densensibilisierungstinte zum Trocken- oder Nassoffsetdruck, Buchdruck oder Flexodruck einer Oberfläche eines chemischen Selbstkopiersatzes, der mindestens zwei aufeinanderliegende Blätter aufweist, deren einander zugewandte Flächen eine elektrophile und die andere eine nucleophile Ueberzugsschicht tragen, die bei örtlicher Druckanwendung eine farbgebende Reaktion erzeugen, dadurch gekennzeichnet, dass die Tinte als aktiven, densensibilisierenden Bestandteil mindestens eine nucleophile Verbindung enthält, welche das Additionsprodukt eines Mono-, Di- oder Polyisocyanats mit einem ethoxylierten und/oder propoxylierten Derivat eines primären oder sekundären Mono-, Di oder Polyamins oder eines Alkanolamins ist, wobei die Hydroxylfunktionalität des Additionsproduktes nach der Alkoxylierung durch Veretherung, Veresterung oder Urethanbildung auf 0,5 bis 2 vermindert wurde.

2. Tinte nach Anspruch 1, dadurch gekennzeichnet, dass die nucleophile Verbindung die Formel

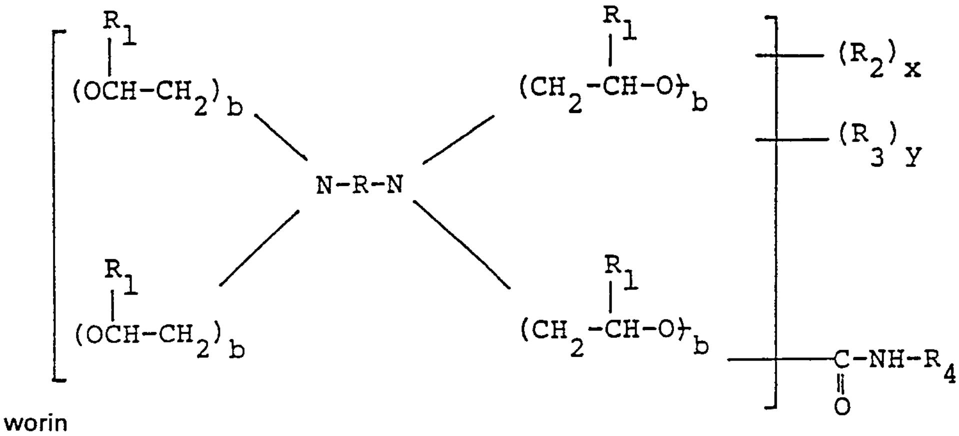

(II)

besitzt, worin

R ein zweiwertiges aliphatisches oder aromatisches Radikal darstellt,

$R_1$ Wasserstoff oder Methyl bedeutet,

$R_2$ eine Alkylgruppe darstellt,

$R_3$ den Acylrest einer $C_2$- bis $C_{22}$-Carbonsäure darstellt,

$R_4$ ein organischer Rest ist, der einen oder mehrere Substituenten der Formel

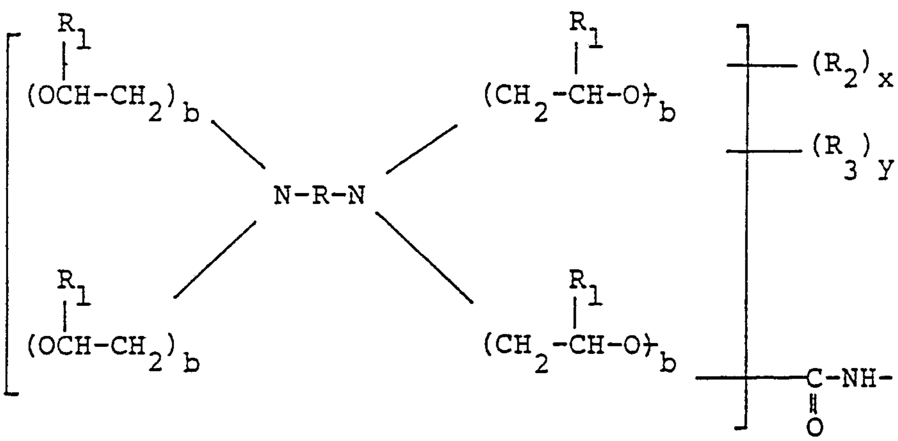

tragen kann,

*x* + *y* den Wert 3 hat, und

*b* eine Zahl von 1 bis 6 darstellt.

3. Tinte nach Anspruch 2, dadurch gekennzeichnet, dass sie eine nucleophile Verbindung der Formel

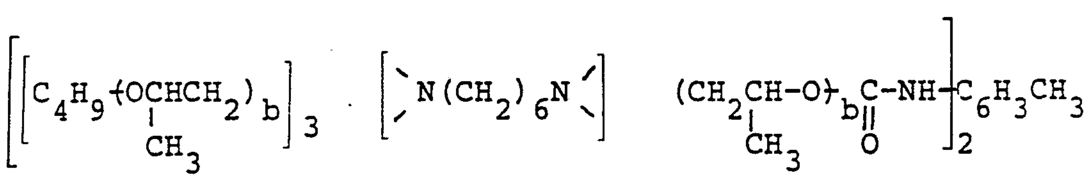

(IV)

enthält, worin *b* 2 oder 3 ist.

4. Tinte nach Anspruch 2, dadurch gekennzeichnet, dass sie eine Verbindung der Formel II enthält, worin R einen Rest der Formel $—(CH_2)_i—$ darstellt, worin *i* eine Zahl von 2 bis 12 ist.

5. Tinte nach Anspruch 2, dadurch gekennzeichnet, dass sie eine Verbindung der Formel II enthält, worin R ein zweiwertiger aromatischer Rest ist.

6. Nucleophile Verbindung zur Verwendung als aktiver Bestandteil in einer Densensibilierungstinte, dadurch gekennzeichnet, dass sie das Additionsprodukt eines Mono-, Di oder Polyisocyanats mit einem, ethoxylierten und/oder propoxylierten Derivat eines primären oder sekundären Mono-, Di- oder Polyamins oder eines Alkanolamins ist, wobei die Hydroxyl-Funktionalität des Additionsproduktes nach dem Alkoxylieren durch Veretherung, Veresterung oder Urethanbildung auf 0,5 bis 2 vermindert wurde.

7. Nucleophile Verbindung nach Anspruch 6, gekennzeichnet durch die Formel

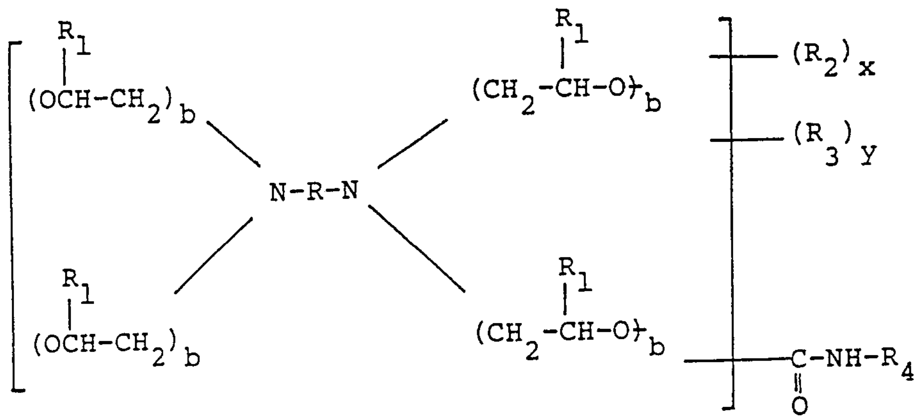

(II)

worin bedeuten:

R einen zweiwertigen aliphatischen oder aromatischen Rest,

$R_1$ Wasserstoff oder Methyl,

$R_2$ eine Alkylgruppe,

$R_3$ den Acylrest einer $C_2$- bis $C_{22}$-Carbonsäure,

$R_4$ einen organischen Rest, der einen oder mehrere Substituenten der Formel

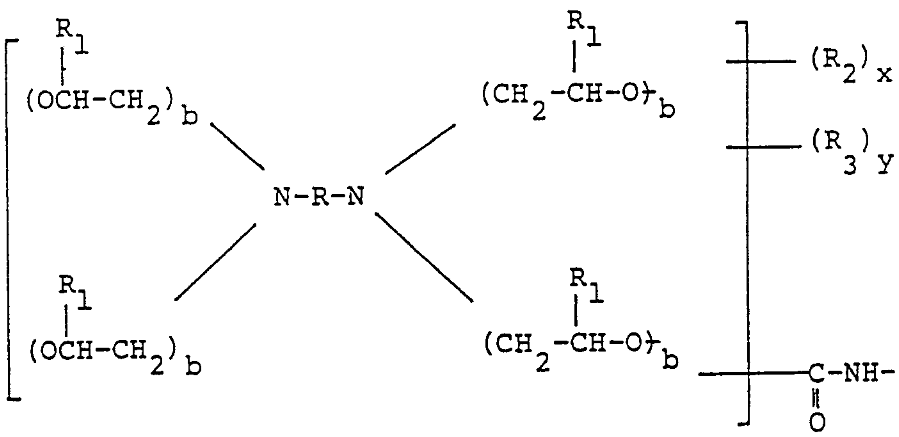

aufweisen kann,

*b* eine Zahl von 1 bis 6 und

$x + y = 3$ bedeuten.

8. Verbindung nach Anspruch 7, dadurch gekennzeichnet, dass sie die Formel

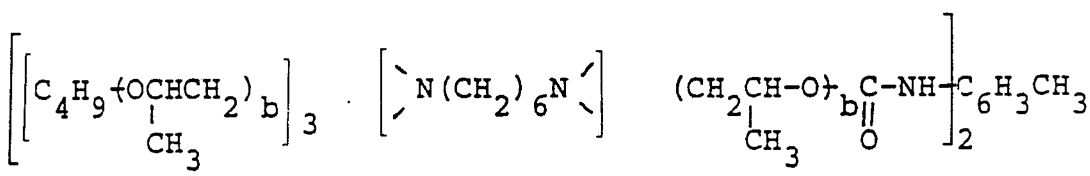

(IV)

besitzt, worin b 2 oder 3 ist.

9. Verbindung nach Anspruch 7, dadurch gekennzeichnet, dass in Formel II R einen Rest der Formel $-(CH_2)_i-$ bedeutet, worin *i* eine Zahl von 2 bis 12 darstellt.

10. Verbindung nach Anspruch 7, dadurch gekennzeichnet, dass in Formel II R einen zweiwertigen aromatischen Rest darstellt.

11. Verfahren zur Herstellung einer Verbindung nach Anspruch 6, dadurch gekennzeichnet, dass man ein primäres oder sekundäres Mono-, Di- oder Polyamin oder ein Alkanolamin ethoxyliert und/oder propoxyliert, dass man die Hydroxyl-Funktionalität durch Veretherung und/oder Acylierung und/oder Urethanbildung mit einem Monoisocyanat auf 0,5 bis 2 vermindert, und dass man das Produkt dann mit einem Mono-, Di- oder Polyisocyanat umsetzt.

12. Verfahren zur Herstellung einer Verbindung der Formel II nach Anspruch 7, dadurch gekennzeichnet, dass man einen Hydroxyether bzw. Hydroxyether der Formel III

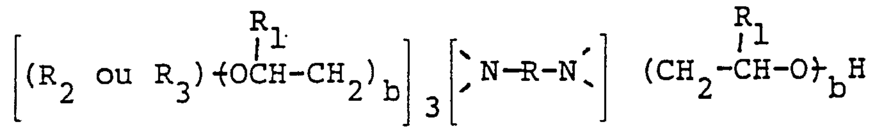

(III)

mit einem Mono-, Di- oder Polyisocyanat umsetzt, welches die Gruppierung $R_4$ aufweist, wobei die Bedeutung der Symbole in Anspruch 7 angegeben ist.

13. Verfahren nach Anspruch 12, dadurch gekennzeichnet, dass man eine Diisocyanat der Formel $(OCN)_2R'$ verwenet, worin R' ein zweiwertiger aromatischer Rest ist.

14. Verfahren nach Anspruch 12, dadurch gekennzeichnet, dass man einen partiellen Ether der Formel

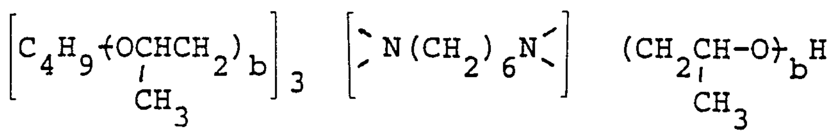

worin *b* 2 oder 3 ist, mit Toluylendiisocyanat umsetzt.

**Claims**

1. A desensitizing ink for dry or humid offset, typographic or flexographic printing on a surface of a chemical autocopying set comprising at least two superimposed sheets whose facing surfaces are covered with an electrophilic layer and as nucleophilic layer, respectively, capable of producing a chromogenic reaction upon the application of local pressure, characterized by the fact that the ink contains an active desensitizing agent comprising at least one nucleophilic compound being the addition product of a mono, di or polyisocyanate to an ethoxylated and/or propoxylated derivative of a primary or secondary mono, di or polyamine or of an alcanolamine, wherein the hydroxyl functionality of the derivative has been reduced after alkoxylation to a value of from 0,5 to 2 by etherification, esterification or urethanisation.

2. The ink of claim 1, characterized by the fact that the nucleophilic compound has the formula

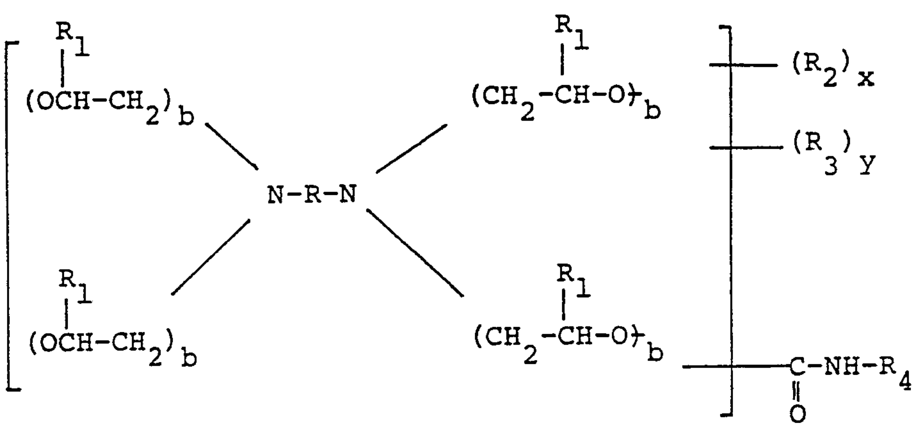

(II)

wherein

R represents a bivalent aliphatic or aromatic radical,

$R_1$ is hydrogen or methyl,

$R_2$ is an alkyl group,

$R_3$ is the acyl radical of a $C_2$ to $C_{22}$ carboxylic acid,

$R_1$ is an organic group which may comprise one or more substituents of the formula

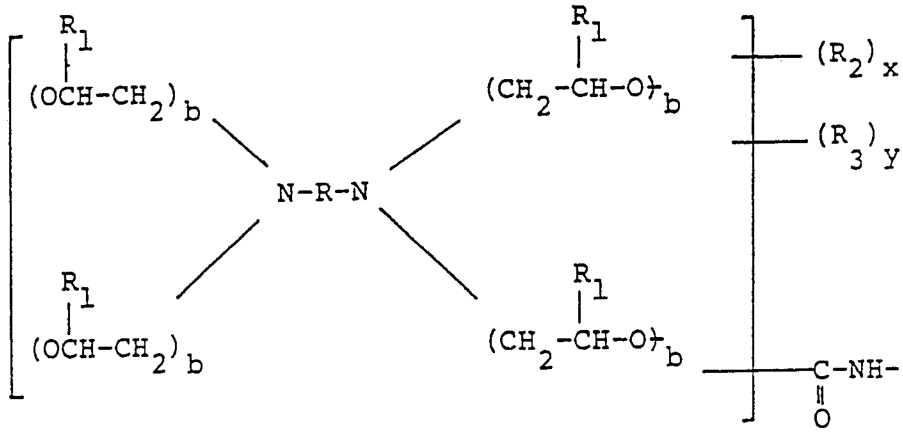

*x* + *y* are 3, and
*b* is a number comprised between 1 and 6.

3. The ink of claim 2, characterized by the fact that it contains a compound of the formula

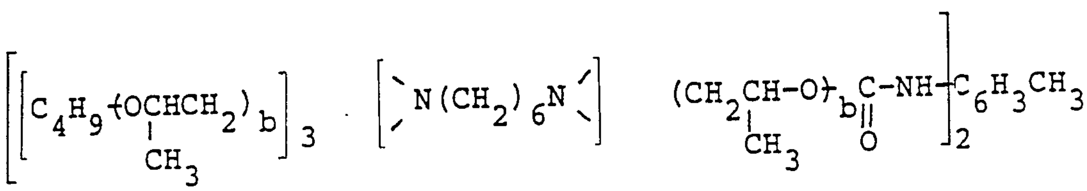

(IV)

wherein *b* is 2 or 3.

4. The ink of claim 2, characterized by the fact that it contains a compound of formula II wherein R is a group of the formula $-(CH_2)_i-$ wherein *i* is a number of from 2 to 12.

5. The ink of claim 2, characterized by the fact that it contains a compound of formula II wherein R is a bivalent aromatic radical.

6. A nucleophilic compound for use as an active agent in a desensitizing ink, characterized by the fact that it is the addition product of a mono, di or polyisocyanate and an ethoxylated and/or propoxylated derivative of a primary or secondary mono, di or polyamine or of an alkanolamine, the hydroxyl functionality of said derivative having been reduced after alkoxylation to a value between 0,5 and 2 by etherification, esterification or urethanisation.

7. The nucleophilic compound of claim 6, characterized by the formula

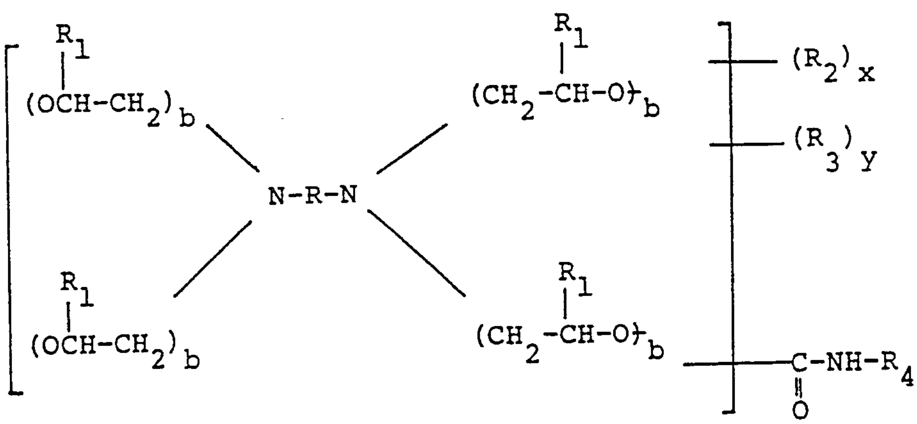

(II)

wherein

R represents a bivalent aliphatic or aromatic radical,

$R_1$ is hydrogen or methyl,

$R_2$ is an alkyl group,

$R_3$ is the acyl radical of a $C_2$ to $C_{22}$ carboxylic acid,

$R_4$ is an organic group which may comprise one or more substituents of the formula

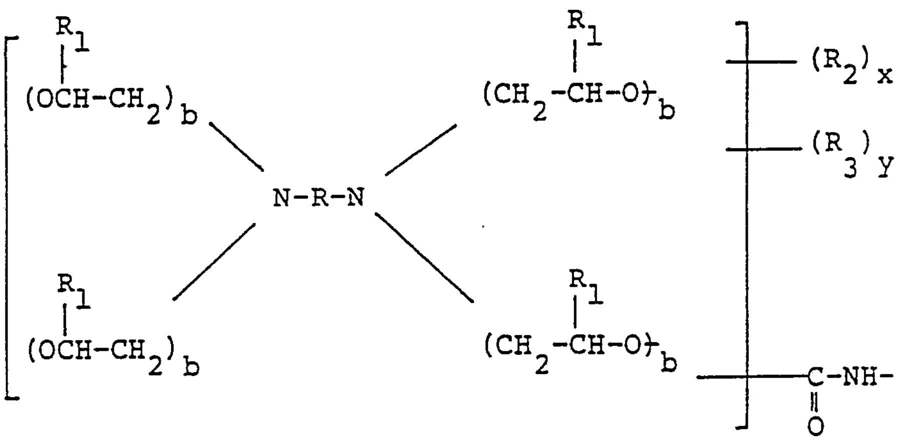

*b* is a number comprised between 1 and 6, and

*x* + *y* is 3.

8. The compound of claim 7, characterized by the fact that it has the formula

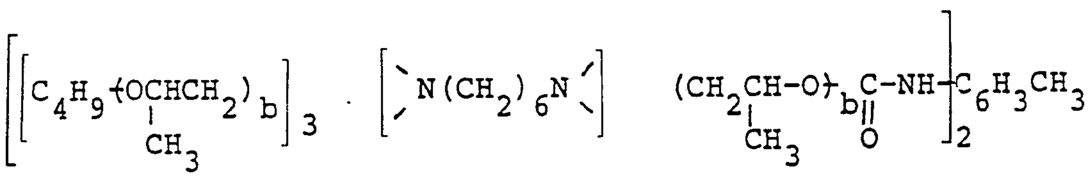

(IV)

wherein b is 2 or 3.

9. The compound of claim 7, characterized by the fact that R in formula II is a radical of formula $-(CH_2)_i-$ wherein *i* is a number of from 2 to 12.

10. The compound of claim 7, characterized by the fact that R in formula II is a bivalent aromatic radical.

11. A process for the preparation of a compound according to claim 6, characterized by the fact that a primary or secondary mono, di or polyamine or an alkanolamine is ethoxylated and/or propoxylated, that the hydroxyl functionality is reduced to a value of 0,5 to 2 by etherification and/or acylation and/or urethanication by means of a monoisocyanate, and the product is reacted with a mono, di or polyisocyanate.

12. A process for the preparation of a compound of formula II according to claim 7, characterized by the fact that a hydroxyether or hydroxyester, respectively, having the formula III

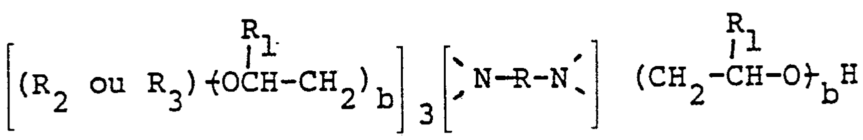

(III)

is reacted with a mono, di or polyisocyanate comprising the group $R_4$, the signification of the symbols being defined in claim 7.

13. The process of claim 12, characterized by the fact that a diisocyanate is used having the formula $(OCN)_2R'$ where R' is a bivalent aromatic radical.

14. The process of claim 12, characterized by the fact that a partial ether of the formula

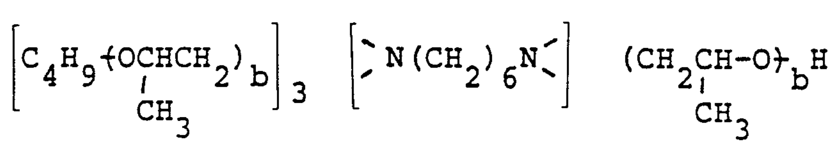

wherein *b* is 2 or 3, is reacted with toluylene diisocyanate.